(19) Europäisches Patentamt
European Patent Office
Office européen des brevets

(11) Publication number: **0 137 717**
**A2**

(12) # EUROPEAN PATENT APPLICATION

(21) Application number: 84306063.3

(22) Date of filing: 05.09.84

(51) Int. Cl.[4]: C 07 D 233/56
C 07 D 249/08, A 01 N 43/50
A 01 N 43/653

(30) Priority: 06.09.83 US 529626

(43) Date of publication of application: 17.04.85 Bulletin 85/16

(84) Designated Contracting States: AT BE CH DE FR GB IT LI LU NL SE

(71) Applicant: E.I. DU PONT DE NEMOURS AND COMPANY
1007 Market Street
Wilmington Delaware 19898(US)

(72) Inventor: Basarab, Gregory Steven
705 W. 28th Street
Wilmington Delaware 19802(US)

(72) Inventor: Boswell, George Albert, Jr.
45 Rockford Road
Wilmington Delaware 19806(US)

(74) Representative: Hildyard, Edward Martin et al,
Frank B. Dehn & Co. Imperial House 15-19 Kingsway
London WC2B 6UZ(GB)

(54) **Fungicidal triazole and imidazole compounds.**

(57) Compounds of the general formula

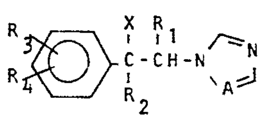

I

wherein A is CH or N;
X is F, Cl or Br;
$R_1$ is H or $C_1-C_4$ alkyl
$R_2$ is alkyl, cycloaklyl or optionally substituted phenyl;
and their salts with protic acids, and complexes with metal ions; exhibit potent fungicidal activity. The compounds may be used for controlling fungal diseases of plants.

The novel compounds may be made by reacting a corresponding starting material wherein X is OH with a suitable halogenating agent.

EP 0 137 717 A2

Croydon Printing Company Ltd.

# FUNGICIDAL TRIAZOLE AND IMIDAZOLE COMPOUNDS

## Background of the Invention

This invention concerns fungicidal triazole and imidazole compounds useful for controlling fungal diseases of plants.

U.S. Patent 4,102,891 discloses compounds of the formula:

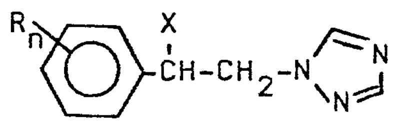

wherein X is halogen and R is halogen, alkyl, alkoxy, alkylthio, alkylsulfonyl, haloalkyl, $NO_2$, CN, phenyl or phenoxy (n=0-3). It is taught that the compounds are useful as fungicides.

U.S. Patent 4,256,754 discloses compounds of the formula:

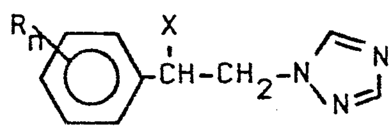

wherein X is hydrogen, halogen, lower alkylmercapto, or lower alkenylmercapto and R is halogen (n=0-2). It is stated that these compounds are useful as insecticides.

EP-A-55,997 discloses fungicidal compounds of the formula:

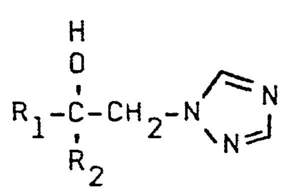

where $R_1$ is alkyl, cycloalkyl, or substituted phenyl; and $R_2$ is substituted phenyl or benzyl.

Belgian Patent 867,245 discloses compounds of the formula:

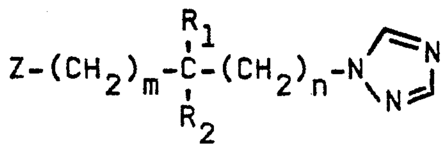

where

Z is aryl;

$R_1$ is CN, H, alkyl, cycloalkyl, alkenyl, cycloalkenyl, alkynyl, substituted aryl, or substituted aralkyl;

$R_2$ is H, alkyl, cycloalkyl, alkenyl, cycloalkenyl, alkynyl, substituted aryl, substituted aralkyl, alkoxy, alkenoxy, alkynoxy, OH, substituted aryloxy, or substituted aralkyloxy;

m is 0 or 1; and

n is 1 or 2.

These compounds are disclosed as agricultural fungicides.

U.S. Patent 4,327,104 discloses compounds of the formula:

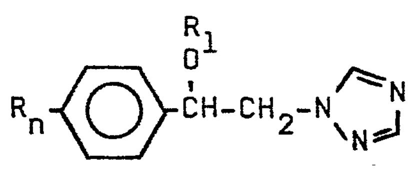

wherein R is halogen, alkyl, alkoxy, alkylthio, alkylsulfonyl, haloalkyl, $NO_2$, CN, phenyl, substituted phenyl, phenoxy or substituted phenoxy (n=0-3), and $R_1$ is alkyl, alkenyl, alkynyl, phenyl, substituted phenyl, benzyl, substituted benzyl, styryl or substituted styryl. These compounds are disclosed as fungicides.

## Summary of the Invention

The present invention provides novel compounds of Formula I, agriculturally useful compositions of the compounds, and the method of using these compounds to control plant fungus diseases.

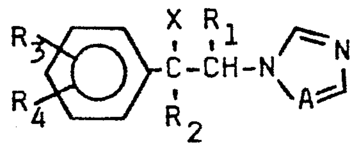

I

wherein

A is CH or N;

X is F, Cl, or Br;

$R_1$ is H or $C_1$-$C_4$ alkyl; and

$R_2$ is $C_1$-$C_6$ alkyl, $C_3$-$C_6$ cycloalkyl,

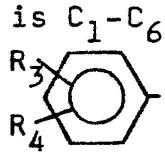 , or 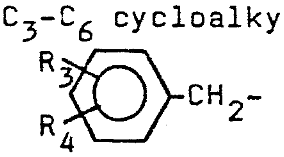

where $R_3$ and $R_4$ are independently H, halogen, $OR_5$, $OCF_3$, $SR_5$, $SO_2R_5$, $CF_3$, $NO_2$, CN, $C_1$-$C_6$ alkyl, phenyl, or phenyl substituted with $C_1$-$C_4$ alkyl, halogen, $CF_3$, or mixtures of these three; and

$R_5$ is $C_1$-$C_6$ alkyl.

Also provided by the present invention are fungicidally active salts of compounds of Formula I with protic acids and complexes of compounds of Formula I with metal ions.

Preferred for their high activity and favorable ease of synthesis are compounds of general Formula I wherein X is fluorine.

More preferred for their higher activity and/or more favorable ease of synthesis are compounds of the preferred scope wherein $R_2$ is 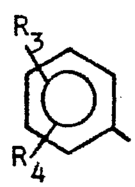 or 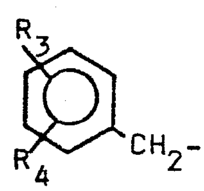 ;

and $R_3$ and $R_4$ are independently H, $CH_3$, $OCH_3$, $OCF_3$, $SCH_3$, $SO_2CH_3$, halogen, or phenyl.

Most preferred for their highest activity and/or most favorable ease of synthesis are compounds of the scope immediately described above wherein $R_3$ and $R_4$ are independently H or halogen, and A is nitrogen.

Specifically preferred for their fungicidal activity and ease of synthesis are:

- 1-[2-fluoro-2,2-bis(4-fluorophenyl)ethyl]-1H-1,2,4-triazole;
- 1-[3-(2,4-dichlorophenyl)-2-fluoro-2-phenylpropyl]-1H-1,2,4-triazole; and
- 1-[2-fluoro-2,2-bis(4-chlorophenyl)ethyl]-1H-1,2,4-triazole.

## Detailed Description of the Invention

### Synthesis

The Formula I compounds of this invention can be prepared by reacting a compound of general formula II,

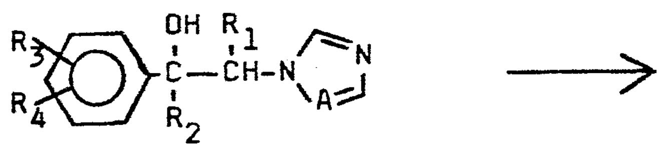

I

II

in which $R_1$-$R_4$ and A are as defined above, as follows. To prepare compounds of Formula I wherein X is fluorine, compounds of Formula II can be reacted with diethylamino sulfur trifluoride. To prepare compounds of Formula I wherein X is chlorine, Formula II compounds can be reacted with thionyl chloride in the presence of a tertiary amine, such as triethylamine, or with triphenylphosphine-carbon tetrachloride. To prepare compounds of Formula I wherein X is bromine, the procedure for the compounds where X = Cl is followed, but with the corresponding bromo reagents, thionyl bromide or triphenylphosphine-carbon tetrabromide.

In these reactions, roughly equimolar amounts of the reactants can be used, although the halogenating reagent is preferably used at a 5-10% molar excess. The reaction is run in an organic solvent. Suitable solvents include non-polar aprotic solvents such as dichloromethane, chloroform, or 1,2-dichloroethane; or ether solvents such as tetrahydrofuran. A polar, inert solvent such as acetonitrile can also be used. The triphenylphosphine reagent can be used in carbon tetrachloride (X = Cl) or in carbon tetrabromide (X = Br) as the solvent.

The reaction temperature can vary between -100°C and 0°C, preferably between -75°C and -20°C, when the halogenating reagent is diethylamino sulfur trifluoride, thionyl chloride, or thionyl bromide. When triphenylphosphine-carbon tetrachloride or triphenylphosphine-carbon tetrabromide are used, the reaction temperature can be about 20°-100°C. As those skilled in the art will recognize, the optimum temperature and reaction time can vary with the exact choice and concentration of reactants and with the choice of solvent. Generally, reaction times of 1-2 hours are required. Reactions are normally conducted at atmospheric pressure but can be conducted under autogenous pressure as well.

The above described reactions provide compounds of Formula I which are frequently contaminated with the by-product of their own dehydrohalogenation. Formula I compounds in which X is fluorine are more readily purified or separated from their by-products by procedures well known in the art and for that reason are preferred.

The intermediate compounds of general Formula II can be prepared by reacting 1,2,4-triazole or imidazole with either compounds of Formula III or Formula IV

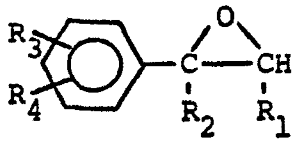

III

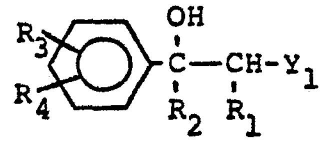

IV

where $Y_1$ is chlorine or bromine and $R_1$, $R_2$, $R_3$, and $R_4$ are as described previously.

The intermediate compounds of Formula II can also be prepared by reacting a compound of Formula V with a Grignard reagent VI

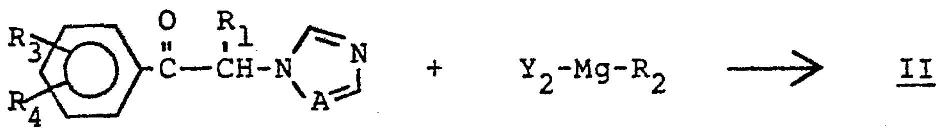

V VI

wherein $R_1$, $R_2$, $R_3$, $R_4$, and A are as defined previously and $Y_2$ is a halogen atom (preferably Cl, Br, or I). This reaction is conducted in an ether solvent such as diethyl ether or tetrahydrofuran at a temperature of between 0°-60°C, depending on the identity of the Grignard reagent and the solvent.

Compounds of Formula V can be obtained by reaction of 1,2,4-triazole or 1,3-imidazole with compounds of general Formula VII

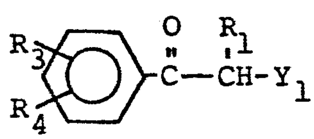

VII

where $R_1$, $R_3$, $R_4$, and $Y_1$ are as defined above [for a general reference to compounds of Formula VII, see J. Med. Chem., 12, 784 (1969)].

Those skilled in the art will recognize that the preparation of the triazole compounds of this invention can produce a mixture of two triazole isomers, the 1H-1,2,4-triazol-1-yl compound and the 4H-1,2,4-triazol-4-yl compound. Since the former isomer will be the predominant one, Formula I is drawn to depict its structure, and the compounds will be referred to as the 1H,1yl triazoles. It is to be recognized, however, that the 4H,4yl isomer can be present, and since this isomer is also fungicidally active, no separation of the isomers is necessary after preparation of the compounds.

It will be further recognized that the Formula I compounds can contain up to two asymmetric carbon atoms (when $R_1$ = $C_1$-$C_4$ alkyl and/or when $R_2$ is different from the substituted phenyl group that is in the ß-position relative to the heterocycle). The stereoisomers that can result all have fungicidal activity, and separation of these isomers is not required.

In the following examples, abbreviations for nuclear magnetic resonance (NMR) spectra are as follows: s = singlet; d = doublet, t = triplet; q = quartet; and m = multiplet. Peak positions for NMR are reported as parts per million downfield from the internal tetramethylsilane standard. Infrared (IR) peak positions are reported in reciprocal centimeters ($cm^{-1}$). Also, unless otherwise stated, ether refers to diethyl ether.

## Example 1

Preparation of 1-[2,2-bis(4-Chlorophenyl)-2-fluoroethyl]-1H-1,2,4-triazole

A solution of 1-[2,2-bis(4-chlorophenyl)-2-hydroxyethyl]-1H-1,2,4-triazole (2.5 g, 7.5 mmol) in 30 ml of dry methylene chloride was cooled to -70°C. A solution of diethylamino sulfur trifluoride (1.3 g, 8.1 mmol) in 5 ml of methylene chloride was added dropwise over a 10-minute period. The solution was

warmed to about 22°C and stirred for 1 hour. Solvent was removed and the residue was chromatographed (ether) on silica gel. Eluted first were less polar by-products followed by the subject compound, which was eluted as a white solid (1.0 gm, 40%): m.p. 187-189°C; $^1H$ NMR ($CDCl_3$): δ 8.2 (d, J = 2Hz, 1H), 7.8 (s, 1H), 7.3 (s, 8H), 5.1 (d, J = 27Hz, 2H).

Example 2

Preparation of 1-[2-(1,1'-Biphenyl-4-yl)-2-fluoro-3-phenylpropyl]-1H-1,2,4-triazole

A solution of 1-[2-(1,1'-biphenyl-4-yl)-2-hydroxy-3-phenylpropyl]-1H-1,2,4-triazole (10.0 g, 28.2 mmol) in 30 ml of dry methylene chloride is cooled to -78°C. A solution of diethylamino sulfur trifluoride (4.9 g, 30.4 mmol) in 20 ml of methylene chloride is added dropwise at such a rate that the temperature remains below -70°C. The solution is warmed to about 22°C and stirred for 1 hour. Solvent is removed and the residue is chromatographed (4:1 methylene chloride:acetone) on silica gel to purify product.

Example 3

Preparation of 1-[2-Fluoro-2,2-bis(4-fluorophenyl)-1-methylethyl]-1H-1,2,4-triazole

A solution of 1,1-bis(4-fluorophenyl)-1-propylene oxide (20.0 gm, 81.3 mmol) and potassium triazole (22 gm, .205 mol) in 200 ml of ethanol was heated at reflux under nitrogen for 40 hours. The solution was poured into water and extracted with ethyl acetate. The extract was washed with water and with brine and dried ($MgSO_4$). Removal of solvent gave a gummy oil which was chromatographed on silica gel (9:1 methylene chloride-acetone) to afford 8.0 gm (31%) of 1-[2,2-bis-(4-fluorophenyl)-2-hydroxy-1-methylethyl]-1H-1,2,4-triazole as a white solid. A portion of this material (4.0 gm, 12.7 mmol) was dissolved in methylene chloride and cooled to -75°C under a nitrogen atmosphere. Diethylaminosulfur trifluoride (1.9 ml, 15.6 mmol) was added dropwise. The solution was warmed to room temperature and solvent was removed. The residue was chromatographed (19:1 methylene chloride-acetone) on silica gel to afford 1.95 gm (48%) of product as a white solid: m.p. 87-88°C. $^1$H NMR ($CDCl_3$): δ 8.1 (d, J=2Hz, 1H), 7.7 (s, 1H), 7.4 (d of d, J = 9,6Hz, 2H), 7.2 (d of d, J = 9,2Hz), 7.1 (t, J = 9Hz, 2H), 6.9 (t, J = 9Hz, 2H), 5.5 (d of q, J = 29, 7Hz), 1.5 (d, J = 7Hz).

## Example 4

### Preparation of 1-[2-Chloro-2-(4-methoxyphenyl)-3,3-dimethylbutyl]-1H-1,2,4-triazole

A solution of 1-[2-hydroxy-2-(4-methoxyphenyl)-3,3-dimethylbutyl]-1H-1,2,4-triazole (10.0 g, 38.0 mmol) and thionyl chloride (4.98 g, 41.9 mmol) in 250 ml dry tetrahydrofuran is cooled to 0°C. Triethylamine (4.5 g, 44.5 mmol) is added dropwise over a 30-minute period, and the solution is then stirred an additional 90 minutes while gradually warming to about 22°C. The solution is filtered and concentrated. The residue is chromatographed on silica gel (ether) to purify product.

## Example 5

### Preparation of 1-(2-Bromo-2-cyclohexyl-2-phenylethyl)-1H-1,2,4-triazole

A solution of 1-(2-cyclohexyl-2-hydroxy-2-phenylethyl)-1H-1,2,4-triazole (10.0 g, 38.9 mmol) and thionyl bromide (8.89 g, 42.8 mmol) in 250 ml dry tetrahydrofuran is cooled to 0°C. Triethylamine (4.5 g, 44.5 mmol) is added dropwise over a 30-minute period and the solution is then stirred an additional 90 minutes while gradually warming to about 22°C. The solution is filtered and concentrated. The residue is chromatographed on silica gel (ether) to purify product.

## Example 6

**Preparation of the 1:1 Complex of 1-[2-Fluoro-2,2-bis-(4-fluorophenyl)ethyl]-1H-1,2,4-triazole and cuprous chloride**

A mixture of 3.0 g (0.01 mol) of 1-[2-fluoro-2,2-bis(4-fluorophenyl)ethyl]-1H-1,2,4-triazole and 1.0 g (0.01 mol) of cuprous chloride in 150 ml of tetrahydrofuran is refluxed under $N_2$ for 30 minutes, and the resulting deep green solution is evaporated to yield the title compound as a green solid, m.p. 75-77°C.

The following additional metal complexes can be prepared similarly using the appropriate metal salt:

1:1 complex with cupric chloride;
2:1 complex with cupric chloride;
1:1 complex with zinc chloride; and
1:1 complex with manganous sulfate.

## Example 7

**Preparation of the 4-Dodecylbenzenesulfonate Salt of 1-[2-Fluoro-2,2-bis(4-fluorophenyl)ethyl]-1H-1,2,4-triazole**

A solution of 3.0 gm (0.01 mol) of 1-[2-fluoro-2,2-bis(4-fluorophenyl)ethyl]-1H-1,2,4-triazole in 30 ml of dichloromethane is combined with a solution of 3.3 g (0.01 mol) of 4-dodecylbenzenesulfonic acid in 30 ml of dichloromethane. The resulting solution is evaporated to yield the title compound as a waxy solid, m.p. 95-104°C.

The following additional acid complexes can be prepared similarly using the appropriate acid.

1:1 complex with hydrochloric acid;
1:1 complex with nitric acid, m.p. 151-152°C; and
1:1 complex with trifluoroacetic acid.

Following the procedures described earlier and exemplified in Examples 1-5, the compounds in Table I can be prepared.

Table I

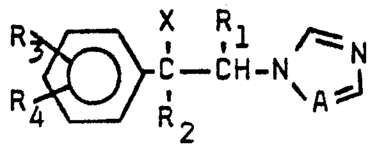

| 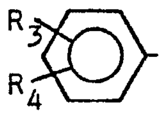 | $R_2$ | $R_1$ | X | A | m.p.(°C) |
|---|---|---|---|---|---|
| 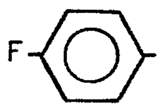 | 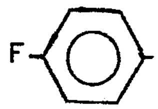 | H | F | N | 120-122° |
| 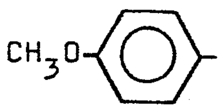 | 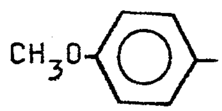 | H | F | N | |
| 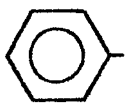 | 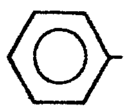 | H | F | N | |
| 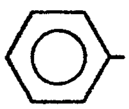 | 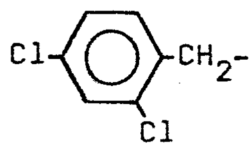 | H | F | N | 88-91° |
| 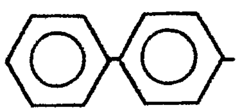 | 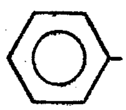 | H | F | N | |
| 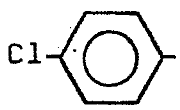 | 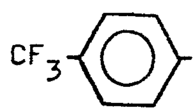 | H | F | N | |
| 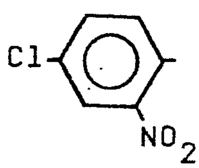 | 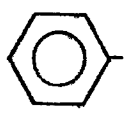 | H | F | N | |
| 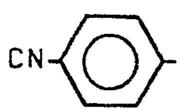 | 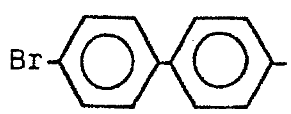 | H | F | N | |
| 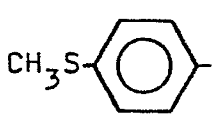 | 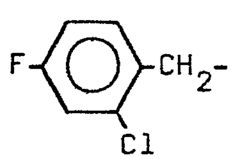 | H | F | N | |

Table I (continued)

| 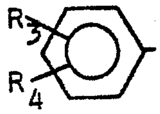 | $R_2$ | $R_1$ | X | A | m.p.(°C) |
|---|---|---|---|---|---|
| 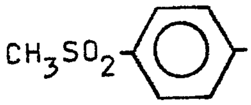 | 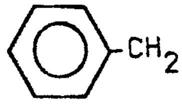 | H | F | N | |
| 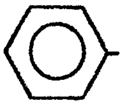 | 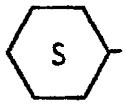 | H | F | N | |
| 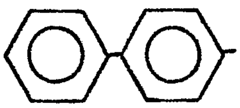 | $CH_3$- | H | F | N | |
| 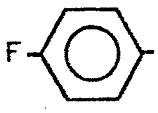 | $CH_3CH_2CH_2CH_2$- | H | F | N | |
| 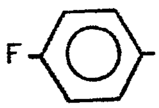 | 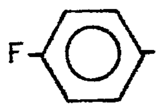 | H | F | CH | |
| 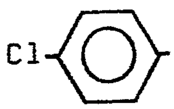 | 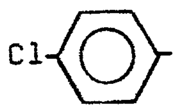 | H | F | CH | |
| 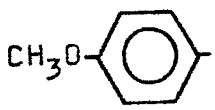 | 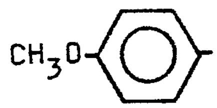 | H | F | CH | |
| 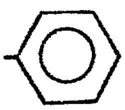 | 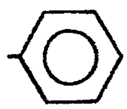 | H | F | CH | |
| 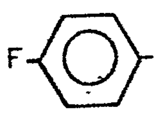 | 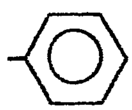 | H | F | CH | |
| 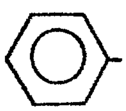 | 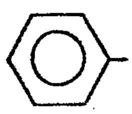 | H | F | CH | |
| 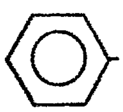 | 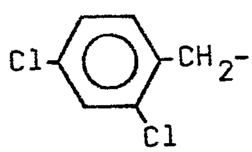 | H | F | CH | |
| 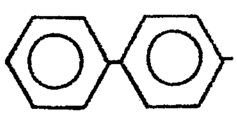 | $CH_3$- | H | F | CH | |

0137717

Table I (continued)

| 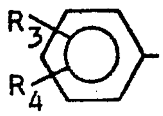 | $R_2$ | $R_1$ | X | A | m.p.(°C) |
|---|---|---|---|---|---|
| 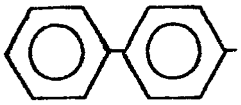 | 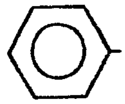 | H | F | CH | |
| 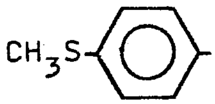 | 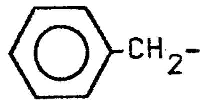 | H | F | CH | |
| 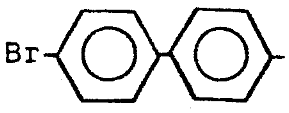 | 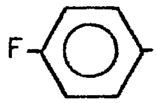 | H | F | CH | |
| 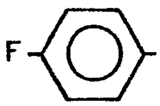 | $CH_3CH_2CH_2CH_2$- | H | F | CH | |
| 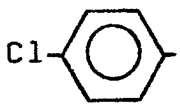 | 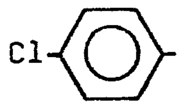 | $CH_3$ | F | N | |
| 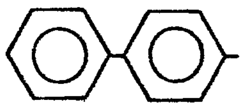 | $CH_3$ | $CH_3$ | F | N | |
| 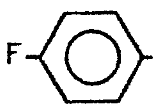 | 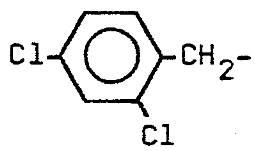 | $CH_3$ | F | N | |
| 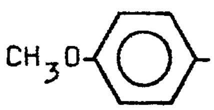 | 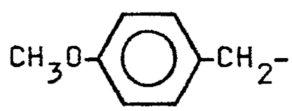 | $CH_3$ | F | N | |
| 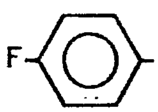 | 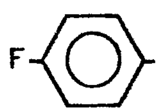 | $CH_3$ | F | CH | |
| 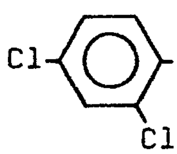 | 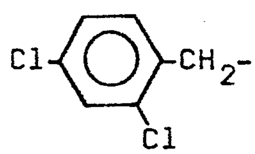 | $CH_3$ | F | CH | |
| 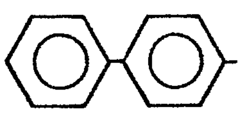 | $CH_3$ | $CH_3$ | F | CH | |

Table I (continued)

| 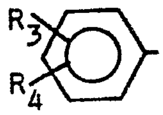 | $R_2$ | $R_1$ | X | A | m.p.(°C) |
|---|---|---|---|---|---|
| 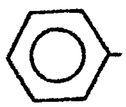 | 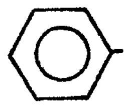 | $\underline{n}$-$C_4H_9$ | F | N | |
| 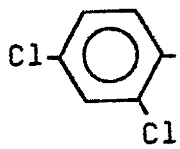 | 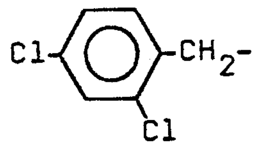 | $\underline{n}$-$C_4H_9$ | F | N | |
| 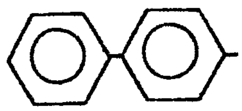 | $\underline{t}$-butyl | $\underline{n}$-$C_4H_9$ | F | N | |
| 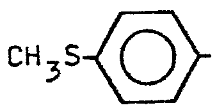 | 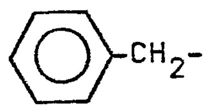 | $\underline{n}$-$C_4H_9$ | F | N | |
| 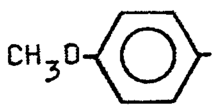 | 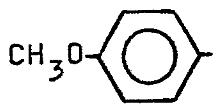 | $\underline{n}$-$C_4H_9$ | F | N | |
| 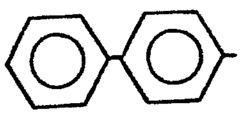 | $CH_3$ | $CH_3$ | Cl | N | |
| 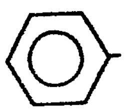 | 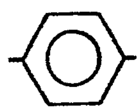 | $CH_3$ | Cl | N | |
| 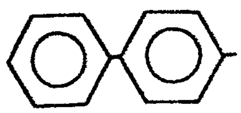 | $CH_3$ | $CH_3$ | Br | N | |
| 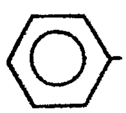 | 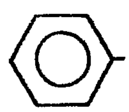 | $CH_3$ | Br | N | |
| 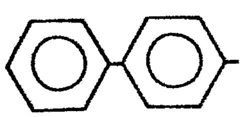 | $CH_3$ | H | Cl | N | |
| 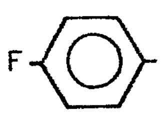 | 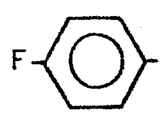 | H | Cl | N | |
| 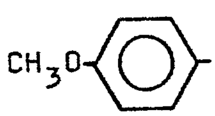 | $\underline{t}$-$C_4H_9$ | H | Cl | N | |

Table I (continued)

| 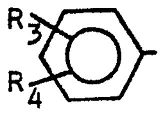 | $R_2$ | $R_1$ | X | A | m.p.(°C) |
|---|---|---|---|---|---|
| 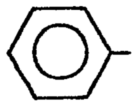 | 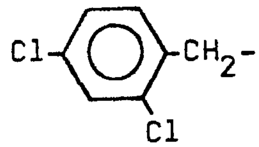 | H | Cl | N | |
| 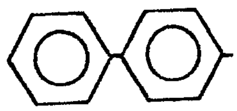 | $CH_3$ | H | Br | N | |
| 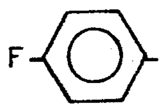 | 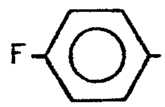 | H | Br | N | |
| 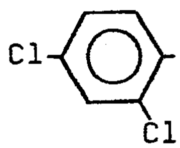 | 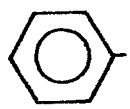 | H | Br | N | |
| 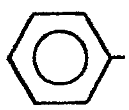 | 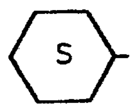 | H | Br | N | |
| 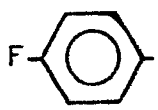 | 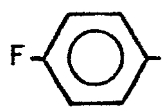 | $C_2H_5$ | F | N | |
| 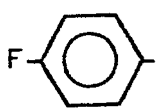 | 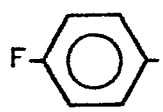 | n-$C_3H_7$ | F | N | |
| 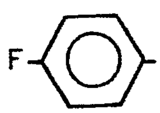 | 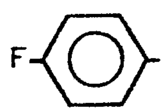 | sec-$C_4H_9$ | F | CH | |
| 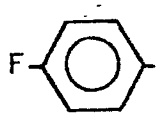 | n-$C_6H_{13}$ | H | F | N | |
| 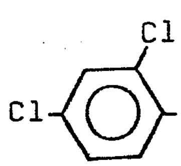 | n-$C_6H_{13}$ | H | F | CH | |
| 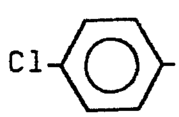 | 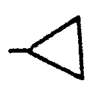 | H | F | N | |

Table I (continued)

| 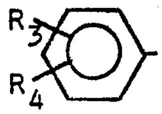 | $R_2$ | $R_1$ | X | A | m.p.(°C) |
|---|---|---|---|---|---|
| 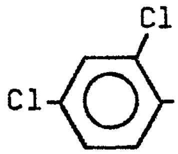 | $CH_3$ | H | F | N | |
| 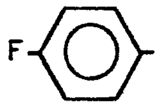 | $CH_3$ | H | F | N | |
| 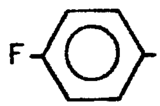 | 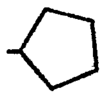 | H | F | N | |
| 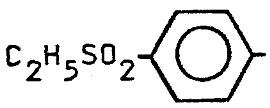 | 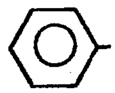 | H | F | N | |
| 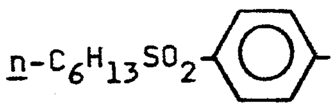 | 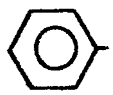 | H | F | CH | |
| 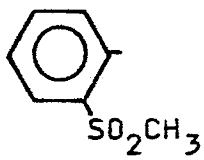 | 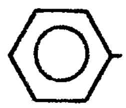 | H | F | N | |
| 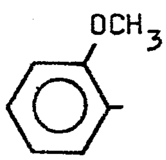 | 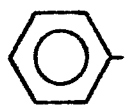 | H | F | N | |
| 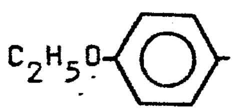 | 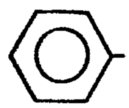 | H | F | CH | |
| 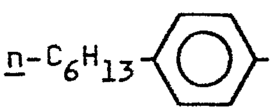 | 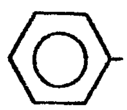 | H | F | N | |
| 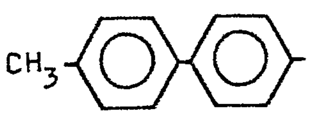 | 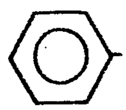 | H | F | CH | |
| 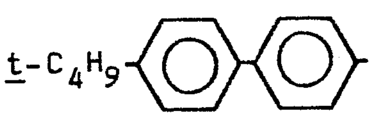 | 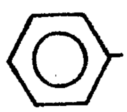 | H | F | N | |

Table I (continued)

| 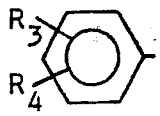 | $R_2$ | $R_1$ | X | A | m.p.(°C) |
|---|---|---|---|---|---|
| 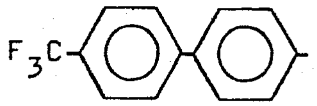 | 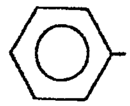 | H | F | N | |
| 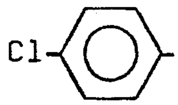 | 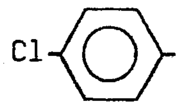 | H | F | N | 186-189° |
| 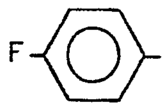 | 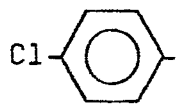 | H | F | N | 141-143° |
| 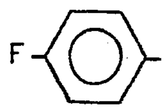 | 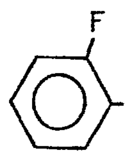 | H | F | N | 137-139° |
| 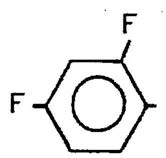 | 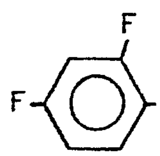 | H | F | N | |

Utility

The compounds of this invention are useful as plant disease control agents. They are effective in controlling a broad spectrum of plant diseases, particularly foliar pathogens of ornamental, vegetable, field, cereal and fruit crops, such as, Puccinia recondita, Sphaerotheca fuliginea, Erysiphe graminis, Podosphaera leucotricha, Venturia inaequalis, Pyricularia oryzae, Bipolaris oryzae, Cercospora arachidicola, Cercospora beticola and Monilinia fructicola. They also control soil borne pathogens such as Rhizoctonia solani.

Formulation and Use

The compounds of this invention will generally be used in formulation with a liquid or solid diluent or with an organic solvent. Useful formulations of the compounds of Formula I can be prepared in conventional ways. They include dusts, granules, pellets, solutions, emulsions, wettable powders, emulsifiable concentrates and the like. Many of these may be applied directly. Sprayable formulations can be extended in suitable media and used at spray volumes of from about one to several hundred liters per hectare. High strength compositions are primarily used as intermediates for further formulation. The formulations, broadly, contain about 1% to 99% by weight of active ingredient(s) and at least one of a) about 0.1% to 20% surfactant(s) and b) about 5% to 99% solid or liquid inert diluent(s). More specifically, they will contain these ingredients in the following approximate proportions:

| | Active Ingredient | Percent by Weight Diluent(s) | Surfactant(s) |
|---|---|---|---|
| Wettable Powders | 20-90 | 0-74 | 1-10 |
| Oil Suspensions, Emulsions, Solutions, (including Emulsifiable Concentrates) | 5-50 | 40-95 | 0-15 |
| Aqueous Suspensions | 10-50 | 40-84 | 1-20 |
| Dusts | 1-25 | 70-99 | 0-5 |
| Granules and Pellets | 1-95 | 5-99 | 0-15 |
| High Strength Compositions | 90-99 | 0-10 | 0-2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey. The more absorptive diluents are preferred for the wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", Allured Publ. Corp., Ridgewood, New Jersey, as well as Sisely and Wood, "Encyclopedia of Surface Active Agents", Chemical Publ. Co., Inc., New York, 1964, list surfactants and recommended uses. All formulations can contain minor amounts of additives to reduce foam, caking, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, Dec. 4, 1967, pp. 147ff. and "Perry's Chemical Engineer's Handbook", 4th Edn., McGraw-Hill, N.Y., 1963, pp. 8-59ff.

For further information regarding the art of formulation, see for example:

H. M. Loux, U.S. Patent 3,235,361, Feb. 15, 1966, Col. 6, Line 16 through Col. 7, Line 19 and Examples 10 through 41.

R. W. Luckenbaugh, U.S. Patent 3,309,192, March 14, 1967, Col. 5, Line 43 through Col. 7, Line 62 and Examples 8, 12, 15, 39, 41, 52, 53, 58, 132, 138-140, 162-164, 166, 167, 169-182.

H. Gysin and E. Knusli, U.S. Patent 2,891,855, June 23, 1959, Col. 3, Line 66 through Col. 5, Line 17 and Examples 1-4.

G. C. Klingman, "Weed Control as a Science", John Wiley and Sons, Inc., New York, 1961, pp. 81-96.

J. D. Fryer and S. A. Evans, "Weed Control Handbook", 5th Edn. Blackwell Scientific Publications, Oxford, 1968, pp. 101-103.

Examples of useful formulations of compounds of the present invention are as follows.

## Example 8

**Wettable Powder**

| | |
|---|---|
| 1-[2-Fluoro-2,2-bis(4-fluorophenyl)ethyl]-1H-1,2,4-triazole | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled, re-blended and packaged.

## Example 9

**High Strength Concentrate**

| | |
|---|---|
| 1-[2-Fluoro-2,2-bis(4-fluorophenyl)ethyl]-1H-1,2,4-triazole | 98.5% |
| silica aerogel | 0.5% |
| synthetic amorphous fine silica | 1.0% |

The ingredients are blended and ground in a hammer-mill to produce a high strength concentrate essentially all passing a U.S.S. No. 50 sieve (0.3 mm openings). This material may then be formulated in a variety of ways.

## Example 10

**Aqueous Suspension**

| | |
|---|---|
| 1-[2-Fluoro-2,2-bis(4-fluorophenyl)ethyl]-1H-1,2,4-triazole | 25% |
| hydrated attapulgite | 3% |
| crude calcium ligninsulfonate | 10% |
| sodium dihydrogen phosphate | 0.5% |
| water | 61.5% |

The ingredients are ground together in a ball, sand, or roller mill until the solid particles have been reduced to diameters under 10 microns.

Example 11

Solution

| | |
|---|---|
| 1-[3-(2,4-Dichlorophenyl)-2-fluoro-2-(4-fluorophenyl)-propyl]-1H-1,2,4-triazole | 30% |
| dimethylformamide | 70% |

The ingredients are combined and stirred to produce a solution, which can be used for low volume applications.

Example 12

Emulsifiable Concentrate

| | |
|---|---|
| 1-[2-Fluoro-2,2-bis(4-fluorophenyl)ethyl]-1H-1,2,4-triazole | 30% |
| blend of oil soluble sulfonates and polyoxyethylene ethers | 4% |
| xylene | 66% |

The ingredients are combined and stirred until the active is dissolved. A fine screen filter is included in packaging operation to insure the absence of any extraneous undissolved material in the product.

Example 13

Granule

| | |
|---|---|
| wettable powder of Example 8 | 15% |
| gypsum | 69% |
| potassium sulfate | 16% |

The ingredients are blended in a rotating or fluid bed mixer and water sprayed on to accomplish granulation. When most of the material has reached the desired range of 1.0 to 0.42 mm. (U.S.S. No. 18 to 40 sieves), the granules are removed, dried, and screened. Oversize material is crushed to produce additional material in the desired range. These granules contain 12% active ingredient.

The compounds of this invention can be mixed with fungicides, bactericides, acaricides, nematicides, insecticides, or other biologically active compounds in order to achieve desired results with a minimum expenditure of time, effort and material. Amounts of these biologically active materials added for each part by weight of the composition of this invention may vary from 0.05 to 25 parts by weight. Suitable agents of this type are well-known to those skilled in the art. Some are listed below:

Fungicides:

methyl 2-benzimidazolecarbamate (carbendazim)
tetramethylthiuram disulfide (thiuram)
*n*-dodecylguanidine acetate (dodine)
manganese ethylenebisdithiocarbamate (maneb)
1,4-dichloro-2,5-dimethoxybenzene (chloroneb)
methyl 1-(butylcarbamoyl)-2-benzimidazolecarbamate (benomyl)
2-cyano-N-ethylcarbamoyl-2-methoxyiminoacetamide (cymoxanil)
N-trichloromethylthiotetrahydrophthalimide (captan)
N-trichloromethylthiophthalimide (folpet)
dimethyl(4,4'-*o*-phenylene)bis(3-thioallophanate) (thiophanate-methyl)
2-(thiazol-4-yl)benzimidazole (thiabendazole)
aluminum tris(O-ethyl phosphonate) ("Aliette")
tetrachloroisophthalonitrile (chlorothalonil)
2,6-dichloro-4-nitroaniline (dichloran)
N-(2,6-dimethylphenyl)-N-(methoxyacetyl)alanine methyl ester (metalaxyl)
cis-N-[(1,1,2,2-tetrachloroethyl)thio]-4-cyclohexene-1,2-dicarbioximide (captafol)
3-(3,5-dichlorophenyl)-N-(1-methyl-ethyl)-2,4-dioxo-1-imidazolidine carboxamide (iprodione)
3-(3,5-dichlorophenyl)-5-ethenyl-5-methyl-2,4-oxazolidinedione (vinclozolin)

kasugamycin

O-ethyl S,S-diphenyl phosphorodithioate (edifenphos)

Bactericides:

tribasic copper sulfate

streptomycin sulfate

oxytetracycline

Acaricides:

senecioic acid, ester with 2-*sec*-butyl-4,6-dinitrophenol (binapacryl)

6-methyl-1,3-dithiolo[2,3-B]quinonolin-2-one (oxythioquinox)

2,2,2-trichloro-1,1-bis(4-chlorophenyl)ethanol (dicofol)

bis(pentachloro-2,4-cyclopentadien-1-yl) (dienochlor)

tricyclohexyltin hydroxide (cyhexatin)

hexakis(2-methyl-2-phenylpropyl)distannoxane (fenbutin oxide)

Nematicides:

2-[diethoxyphosphinylimino]-1,3-dithietane (fosthietan)

S-methyl-1-(dimethylcarbamoyl)-N-(methylcarbamoyloxy)-thioformimidate (oxamyl)

S-methyl-1-carbamoyl-N-(methylcarbamoyloxy)thioformimidate

N-isopropylphosphoramidic acid, O-ethyl-O'-[4-(methylthio)-*m*-tolyl]diester (fenamiphos)

Insecticides:

3-hydroxy-N-methylcrotonamide(dimethylphosphate)ester (monocrotophos)

methylcarbamic acid, ester with 2,3-dihydro-2,2-dimethyl-7-benzofuranol (carbofuran)

O-[2,4,5-trichloro-α-(chloromethyl)benzyl]phosphoric acid, O',O'-dimethyl ester (tetrachlorvinphos)

2-mercaptosuccinic acid, diethyl ester, S-ester with thionophosphoric acid, dimethyl ester (malathion)

phosphorothioic acid, O,O-dimethyl, O-*p*-nitrophenyl ester (methyl parathion)

methylcarbamic acid, ester with α-naphthol (carbaryl)

methyl N-[[(methylamino)carbonyl]oxy]ethanimidothioate (methomyl)

N'-(4-chloro-o-tclyl)-N,N-dimethylformamidine (chlordimeform)

O,O-diethyl-O-(2-isopropyl-4-methyl-6-pyrimidyl)phosphorothioate (diazinon)

octachlorocamphene (toxaphene)

O-ethyl O-p-nitrophenyl phenylphosphonothioate (EPN)

cyano(3-phenoxyphenyl)-methyl 4-chloro-α-(1-methylethyl)benzeneacetate (fenvalerate)

(3-phenoxyphenyl)methyl (±)-cis,trans-3-(2,2-dichloroethenyl)-2,2-dimethylcyclopropanecarboxylate (permethrin)

dimethyl N,N'-[thiobis](N-methylimino)carbonyloxy]]-bis[ethanimidothioate] (thiodicarb)

phosphorothiolothionic acid, O-ethyl-O-[4-(methylthio)-phenyl]-S-n-propyl ester (sulprofos)

α-cyano-3-phenoxybenzyl 3-(2,2-dichlorovinyl)-2,2-dimethylcyclopropane carboxylate (cypermethrin)

cyano(3-phenoxyphenyl)methyl 4-(difluoromethoxy)-α-(methylethyl)benzeneacetate ("Payoff")

O,O-diethyl-O-(3,5,6-trichloro-2-pyridyl)phosphorothioate (chlorpyrifos)

O,O-dimethyl-S-[(4-oxo-1,2,3-benzotriazin-3-(4H)-yl)-methyl]phosphorodithioate (azinphos-methyl)

5,6-dimethyl-2-dimethylamino-4-pyrimidinyl dimethyl carbamate ("Pirimor")

S-(N-formyl-N-methylcarbamoylmethyl-O,O-dimethyl phosphorodithioate (formothion)

S-2-(ethylthioethyl)-O,O-dimethyl phosphiorothioate (demeton-S-methyl)

α-cyano-3-phenoxybenzyl cis-3-(2,2-dibromovinyl)-2,2-dimethylcyclopropane carboxylate (deltamethrin)

cyano(3-phenoxyphenyl)methyl ester of N-(2-chloro-4-trifluoromethylphenyl)alanine ("Mavrik")

Application

Disease control is ordinarily accomplished by applying an effective amount of the compound, normally as part of a formulation containing it, either pre- or post-infection to the portion of the plant to be protected, such as the roots, stems, foliage, fruit, seeds, tubers or bulbs, or to the medium (soil or sand) in which the plants to be protected are growing. The compound may also be applied to the seed from which the plants to be protected are to be grown.

Rates of application for these compounds can be influenced by many factors of the environment and should be determined under actual use conditions. Nevertheless, foliage can normally be protected when treated at a rate of from less than 1 to 500 grams of active ingredient per hectare. Plants growing in soil treated at a concentration from about 0.1 to about 20 kg of active ingredient per hectare can be protected from disease. Seed and seedlings can normally be protected when seed is treated at a rate of from about 0.06 to about 3 grams of active ingredient per kilogram of seed.

In the following Examples 14-28, which further illustrate the present invention, the test compound is 1-[2-fluoro-2,2-bis(fluorophenyl)ethyl]-1H-1,2,4-triazole. Other compounds tested are specifically listed where appropriate.

Example 14

The test compound was dissolved in acetone in an amount equal to 6% of the final volume and then suspended at a concentration of 100 ppm in purified water containing 250 ppm of the surfactant TREM 014 (polyhydric alcohol esters). This suspension was sprayed to the point of run-off on wheat seedlings. The following day, the plants were inoculated with a spore suspension of Puccinia recondita var. tritici, causal agent of wheat leaf rust, and incubated in a saturated humidity chamber at 20° for 24 hours and then in a growth room for an additional 7 days, when disease ratings were made. Disease control was shown to be 100%. Treated plants had no rust pustules while the untreated plants had numerous rust pustules on each leaf.

Using the test conditions described in Example 14, 1-[3-(2,4-dichlorophenyl)-2-fluoro-2-phenylpropyl]-1H-1,2,4-triazole and 1-[2-fluoro-2,2-bis(4-chlorophenyl)ethyl]-1H-1,2,4-triazole gave 100 and 90% control of Puccinia recondita var. tritici, respectively.

Example 15

The test compound was dissolved in acetone in an amount equal to 6% of the final volume and then suspended at a concentration of 100 ppm in purified water containing 250 ppm of the surfactant TREM 014 (polyhydric alcohol esters). This suspension was sprayed to the point of run-off on cucumber seedlings. The following day, the plants were inoculated with a spore suspension of the fungus Sphaerotheca fuliginea, causal agent of cucumber powdery mildew, and incubated in a growth room for 7 days. Disease ratings were then made. Disease control was shown to be 100%. Treated plants had no powdery mildew in contrast to untreated plants which were covered with powdery mildew.

Using the test conditions described in Example 15, 1-[3-(2,4-dichlorophenyl)-2-fluoro-2-phenylpropyl]-1H-1,2,4-triazole and 1-[2-fluoro-2,2-bis(4-chlorophenyl)ethyl]-1H-1,2,4-triazole also gave 100% control of Sphaerotheca fuliginea.

Example 16

The test compound was dissolved in acetone in an amount equal to 6% of the final volume and then suspended in purified water containing 250 ppm of the surfactant TREM 014 (polyhydric alcohol esters). This suspension was sprayed at 935 liters/hectare at a rate of 70 grams/hectare on small apple trees. The following day, the plants were inoculated with a spore suspension of the fungus, Podosphaera leucotricha, causal agent of apple powdery mildew, and incubated in a growth room for 7-10 days. Disease ratings were then made. Disease control was 100%. Treated plants had no powdery mildew in contrast to untreated plants which were covered with powdery mildew.

Example 17

The test compound was dissolved in acetone in an amount equal to 6% of the final volume and then suspended at a concentration of 100 ppm in purified water containing 250 ppm of the surfactant TREM 014 (polyhydric alcohol esters). This suspension was sprayed to the point of run-off on apple seedlings. The following day, the plants were inoculated with a spore suspension of the fungus Venturia inaequalis, causal agent of apple scab, and incubated in a saturated humidity chamber at 20° for 24 hours and then in a growth room for an additional 10-12 days. Disease ratings were then made. Disease control was shown to be 100%. Treated plants had no apple scab lesions

when compared to untreated plants which were covered with scab lesions.

Using the test conditions described in Example 17, 1-[3-(2,4-dichlorophenyl)-2-fluoro-2-phenylpropyl]-1H-1,2,4-triazole and 1-[2-fluoro-2,2-bis(4-chlorophenyl)ethyl]-1H-1,2,4-triazole gave 90 and 100% control of _Venturia inaequalis_, respectively.

Example 18

The test compound was dissolved in acetone in an amount equal to 6% of the final volume and then suspended at a concentration of 100 ppm in purified water containing 250 ppm of the surfactant TREM 014 (polyhydric alcohol esters). This suspension was sprayed to the point of run-off on rice seedlings. The following day, the plants were inoculated with a spore suspension of _Bipolaris oryzae_, causal agent of rice brown leaf spot, and incubated in a saturated humidity chamber at 20° for 24 hours and then in a growth room for an additional 7 days, after which disease ratings were made. Disease control was shown to be 94%. Treated plants had few or no lesions while the untreated plants had numerous lesions on each leaf.

Using the test conditions described in Example 18, 1-[3-(2,4-dichlorophenyl)-2-fluoro-2-phenylpropyl]-1H-1,2,4-triazole and 1-[2-fluoro-2,2-bis(4-chlorophenyl)ethyl]-1H-1,2,4-triazole gave 50 and 60% control of _Bipolaris oryzae_, respectively.

Example 19

The test compound was dissolved in acetone in an amount equal to 6% of the final volume and then suspended at a concentration of 100 ppm in purified water containing 250 ppm of the surfactant TREM 014 (polyhydric alcohol esters). This suspension was sprayed

to the point of run-off on peanut seedlings. The following day, the plants were inoculated with a spore suspension of Cercospora arachidicola, causal agent of peanut early leafspot, and incubated in a saturated humidity chamber at 27° for 24 hours and then in a growth room for an additional 14 days, after which disease ratings were made. Disease control was shown to be 100%. Treated plants had no leafspots while the untreated plants had numerous leafspots.

Using the test conditions described in Example 19, 1-[3-(2,4-dichlorophenyl)-2-fluoro-2-phenylpropyl]-1H-1,2,4-triazole and 1-[2-fluoro-2,2-bis(4-chlorophenyl)ethyl]-1H-1,2,4-triazole gave 90 and 100% control of Cercosporidium personatum, respectively.

## Example 20

The test compound was dissolved in an amount equal to 6% of the final volume and then suspended in purified water containing 250 ppm of the surfactant TREM 014 (polyhydric alcohol esters). This suspension was sprayed at 374 liters/hectare at a rate of 70 grams/hectare on small sugar beet plants. The following day, the plants were inoculated with a spore suspension of the fungus Cercospora beticola, causal agent of sugar beet leafspot, and incubated in a humidity chamber at 30°C for 7-10 days. Disease ratings were then made. Disease control was shown to be 100%. Treated plants had no leafspots when compared to untreated plants which had numerous leafspots.

## Example 21

The test compound was dissolved in acetone in an amount equal to 5% of the final volume and then suspended at a concentration of 100 ppm in purified water

containing 700 ppm of the surfactant TREM 014 (polyhydric alcohol esters). Canned peach halves were dipped in this suspension for three minutes and then placed to air dry in sterile containers. Upon drying, the peach halves were inoculated with two pieces of Monilinia fructicola mycelium, causal agent of stone fruit brown rot, and incubated in the sterile containers for five days. At that time the radii of the colonies' growth were measured on each peach. Colonies on treated peaches did not grow while those growing on untreated peaches covered the entire surface of the peach. Disease control (percent growth inhibition of colonies on treated peaches as compared to that of colonies on untreated peaches) was 100%.

Example 22

The test compound was dissolved in acetone in an amount equal to 6% of the final volume and then suspended at a concentration of 100 ppm in purified water containing 250 ppm of the surfactant TREM 014 (polyhydric alcohol esters). This suspension was sprayed to the point of run-off on rice seedlings. The following day, the plants were inoculated with a mixture of bran and the mycelium of the fungus Rhizoctonia solani, causal agent of sheath blight of rice, and incubated in a growth room for 7 days. Disease ratings were then made. Disease control was shown to be 80%. Treated plants had little sheath blight in contrast to untreated plants which were covered with sheath blight.

## Example 23

The test compound was dissolved in acetone so that 1 ml of solution yielded a concentration of 0.5 kilogram/hectare when added to cotton seeds and soil in pots. Seeds and soil were then inoculated with a mixture of sand, cereal and mycelium of the fungus *Rhizoctonia solani*, causal agent of cotton blight, and incubated in a growth room for 14 days. Disease ratings were then made. Disease control was shown to be 89%. Treated seeds germinated well and had little blight in contrast to the untreated plants which failed to germinate or were blighted shortly after germination.

## Example 24

The test compound was dissolved in acetone so that 1 ml of solution yielded a concentration of 0.1 gram/kilogram of seed when evenly coated on 5 grams of wheat seed. Treated seeds were air dried, planted, incubated in a growth room for 14 days, then inoculated with a spore suspension of the fungus *Puccinia recondita* var. *tritici*, causal agent of wheat leaf rust. Inoculated plants were incubated in a saturated humidity chamber at 20°C for 24 hours and then in a growth room for an additional 7 days, when disease ratings were made. Disease control was shown to be 100%. Plants grown from treated seeds had no rust pustules while plants from untreated seed had numerous rust pustules on each leaf.

## Example 25

The test compound was dissolved in acetone so that 1 ml of solution yielded a concentration of 0.1 gram/kilogram of seed when evenly coated on 5 grams of wheat seed. Treated seeds were air dried, planted,

incubated in a growth room for 14 days, then inoculated with a spore suspension of the fungus *Erysiphe graminis* var. *tritici*, causal agent of wheat powdery mildew, and then further incubated in a growth room for 7 days. Disease ratings were then made. Disease control was shown to be 100%. Plants grown from treated seeds had no powdery mildew in contrast to plants from untreated seed which were covered with powdery mildew.

## Example 26

The test compound was dissolved in acetone so that 1 ml of solution yielded a concentration of 0.1 gram/kilogram of seed when evenly coated on 5 grams of barley seed. Treated seeds were air dried, planted, incubated in a growth room for 14 days, then inoculated with a spore suspension of the fungus *Erysiphe graminis* var. *hordei*, causal agent of barley powdery mildew, and then further incubated in a growth room for 7 days. Disease ratings were then made. Disease control was shown to be 100%. Plants grown from treated seeds had no powdery mildew in contrast to plants from untreated seed which were covered with powdery mildew.

## Example 27

The test compound was dissolved in acetone so that 1 ml of solution yielded a concentration of 0.5 gram/kilogram of seed when evenly coated on 5 grams of wheat seed. Treated seeds were air dried, planted, incubated in a growth room for 7 days, then inoculated with a spore suspension of the fungus *Bipolaris oryzae*, causal agent of rice brown leafspot, and then further incubated in a growth room for 7 days. Disease ratings were then made. Disease control was shown to

be 91%. Plants grown from treated seeds had little or no lesions in contrast to plants from untreated seed which had numerous lesions on each leaf.

Example 28

The test compound was dissolved in acetone in an amount equal to 6% of the final volume and then suspended at a concentration of 100 ppm in purified water containing 250 ppm of the surfactant TREM 014 (polyhydric alcohol esters). This suspension was sprayed to the point of run-off on rice seedlings. The following day, the plants were inoculated with a spore suspension of *Pyricularia oryzae*, causal agent of rice blast, and incubated in a saturated humidity chamber at 28° for 24 hours and then in a growth room for an additional 7 days, after which disease ratings were made. Using as test compounds 1-[3-(2,4-dichlorophenyl)-2-fluoro-2-phenylpropyl]-1H-1,2,4-triazole and 1-[2-fluoro-2,2-bis(4-chloro-phenyl)ethyl]-1H-1,2,4-triazole gave 90 and 60% control of *Pyricularia oryzae*, respectively.

Claims: BA-8542

1. A compound of Formula I:

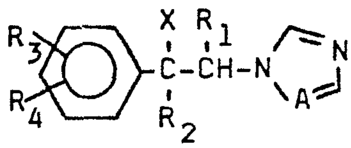

I

wherein

A is CH or N;

X is F, Cl, or Br;

$R_1$ is H or $C_1$-$C_4$ alkyl; and

$R_2$ is $C_1$-$C_6$ alkyl, $C_3$-$C_6$ cycloalkyl,

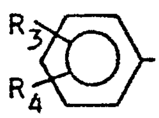 , or 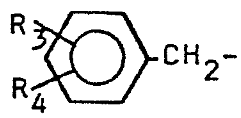

where $R_3$ and $R_4$ are independently H, halogen, $OR_5$, $OCF_3$, $SR_5$, $SO_2R_5$, $CF_3$, $NO_2$, CN, $C_1$-$C_6$ alkyl, phenyl, or phenyl substituted with $C_1$-$C_4$ alkyl, halogen, $CF_3$, or mixtures of these three; and

$R_5$ is $C_1$-$C_6$ alkyl;

and fungicidally active salts of said compound with protic acids, and complexes of said compound with metal ions.

2. A compound of Claim 1 wherein X is fluorine.

3. A compound of Claim 2 wherein

$R_2$ is 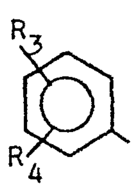 or 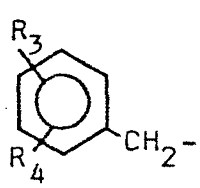 ; and

$R_3$ and $R_4$ are independently H, $CH_3$, $OCH_3$, $OCF_3$, $SCH_3$, $SO_2CH_3$, halogen, or phenyl.

4. A compound of Claim 3 wherein $R_3$ and $R_4$ are independently H or halogen.

5. A compound of Claim 4 wherein A is nitrogen.

6. The compound of claim 5 that is 1-[2-fluoro-2,2-bis(4-fluorophenyl)ethyl]-1H-1,2,4-triazole and fungicidally active salts of said compound with protic acids, and complexes of said compound with metal ions.

7. The compound of claim 5 that is 1-[3-(2,4-dichlorophenyl)-2-fluoro-2-phenylpropyl]-1H-1,2,4-triazole and fungicidally active salts of said compound with protic acids, and complexes of said compound with metal ions.

8. The compound of claim 5 that is 1-[2-fluoro-2,2-bis(4-chlorophenyl)ethyl]-1H-1,2,4-triazole and fungicidally active salts of said compound with protic acids, and complexes of said compound with metal ions.

9. A composition for controlling fungus disease in plants which comprises an effective amount of a compound of any of claims 1 to 8 and at least one of the following: surfactant; solid inert diluent; or liquid inert diluent.

10. A method for controlling fungus disease in plants which comprises applying to the locus of infestation to be protected an effective amount of a compound of any of claims 1 to 8.

11. A process for the preparation of a compound of claim 1 which comprises reacting a compound of general formula

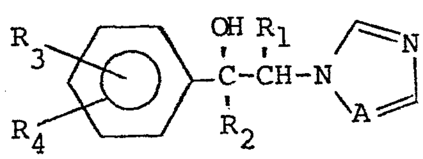

wherein $R_1$, $R_3$, $R_4$ and A are as defined in claim 1, with a suitable halogenating agent, whereafter if desired the product of formula (I) is reacted with a protic acid to form a salt or with a metal compound to form a complex with a metal ion.

BA-8542

Claims:

1. A process for the preparation of a compound of Formula I:

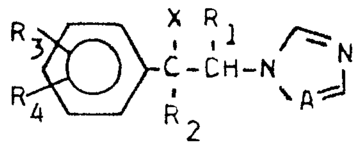

I

wherein

A is CH or N;

X is F, Cl, or Br;

$R_1$ is H or $C_1$-$C_4$ alkyl; and

$R_2$ is $C_1$-$C_6$ alkyl, $C_3$-$C_6$ cycloalkyl,

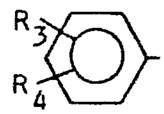 , or 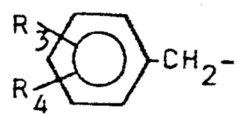

where $R_3$ and $R_4$ are independently H, halogen, $OR_5$, $OCF_3$, $SR_5$, $SO_2R_5$, $CF_3$, $NO_2$, CN, $C_1$-$C_6$ alkyl, phenyl, or phenyl substituted with $C_1$-$C_4$ alkyl, halogen, $CF_3$, or mixtures of these three; and

$R_5$ is $C_1$-$C_6$ alkyl;

or a fungicidally active salt of said compound with a protic acid, or a complex of said compound with a metal ion, which comprises reacting a corresponding compound of general formula

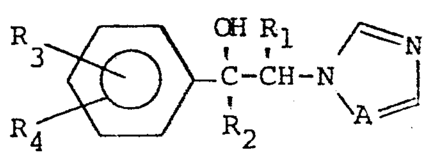

wherein $R_1$, $R_3$, $R_4$ and A are as defined above, with a suitable halogenating agent, whereafter if desired the product of formula (I) is reacted with a protic acid to form a salt or with a metal compound to form a complex with a metal ion.

2. A process of claim 1 wherein said halogenating agent is selected from diethylamino sulfur trifluoride, thionyl chloride or bromide, triphenylphosphine-carbon tetrachloride or triphenyl phospine-carbon tetrabromide.

3. A process of claim 1 or 2 wherein X is fluorine.

4. A process of claim 3 wherein

$R_2$ is 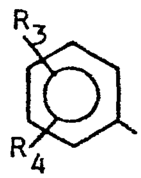 or 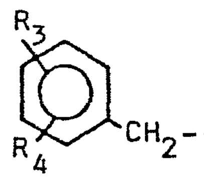 ; and

$R_3$ and $R_4$ are independently H, $CH_3$, $OCH_3$, $OCF_3$, $SCH_3$, $SO_2CH_3$, halogen, or phenyl.

5. A process of claim 4 wherein $R_3$ and $R_4$ are independently H or halogen.

6. A process of claim 5 wherein A is nitrogen.

7. A process of claim 1 wherein the product is a compound selected from:
1-[2-fluoro-2,2-bis(4-fluorophenyl)ethyl]-1H-1,2,4-triazole;
1-[3-(2,4-dichlorophenyl)-2-fluoro-2-phenylpropyl]-1H-1,2,4-triazole;
1-[2-fluoro-2,2-bis(4-chlorophenyl)ethyl]-1H-1,2,4-triazole;
and fungicidally active salts of said compounds with protic acids, and complexes of said compounds with metal ions.

8. A composition for controlling fungus disease in plants which comprises an effective amount of a compound of formula (I) as defined in any of claims 1 to 7 and at least one of the following: surfactant; solid inert diluent; or liquid inert diluent.

9. A method for controlling fungus disease in plants which comprises applying to the locus of infestation to be protected an effective amount of a compound of formula (I) as defined in any of claims 1 to 7.